Europäisches Patentamt

European Patent Office

Office européen des brevets

(19)

(11) Publication number: **0 087 374**
**B1**

(12) **EUROPEAN PATENT SPECIFICATION**

(45) Date of publication of patent specification: 27.05.87

(21) Application number: 83400376.6

(22) Date of filing: 23.02.83

(51) Int. Cl.⁴: **A 23 J 3/00**, A 61 K 7/48

(54) Allantoin-hydrolyzed animal protein product.

(30) Priority: 24.02.82 US 351722
01.06.82 US 383404
13.12.82 US 449117

(43) Date of publication of application:
31.08.83 Bulletin 83/35

(45) Publication of the grant of the patent:
27.05.87 Bulletin 87/22

(84) Designated Contracting States:
AT BE CH DE FR GB IT LI LU NL SE

(56) References cited:
FR-A-2 510 563
US-A-3 941 722

SOAP, PERFUMERY AND COSMETICS, vol. 49,
no. 11, November 1976, pages 481-485. S. B.
MECCA: "Uric acid, allantoin and allantoin
derivatives"

SEIFEN- ÖLE - FETTE - WACHSE, vol. 97, no. 15,
1971, pages 533-534. S. B. MECCA: "Neue
Allantoin-Derivate für die kosmetische und
dermatologische Anwendung"

(73) Proprietor: CHARLES OF THE RITZ GROUP LTD.
40 West 57th Street
New York, NY 10019 (US)

(72) Inventor: Puchalski, Eugene
129 McAdoo Avenue
Jersey City New Jersey (US)
Inventor: Deckner, George E.
645 Horst Street
Westfield New Jersey (US)
Inventor: Dixon, Richard P.
23 Avondale Lane
Aberdeen New Jersey (US)
Inventor: Donahue, Frances A.
2 Kimberley Court Apt. 16
Middletown New Jersey (US)

(74) Representative: Maiffret, Bernard et al
Law Offices of William J. Rezac 49, avenue
Franklin D. Roosevelt
F-75008 Paris (FR)

Courier Press, Leamington Spa, England.

### Description

The present invention relates to a stable form of allantoin which may be used in various skin preparations and other products in amounts of at least 0.5% by weight allantoin, without the allantoin crystallizing out of solution.

Allantoin (glyoxyl diureide) and derivatives thereof such as Alpantha (Schuylkill Chemical Co., Philadelphia, Pa. trademark for "Allantoin DL-Panthenol Modified") are known for their soothing, skin softening and healing activity and have been used in 0.2% concentrations in creams, lotions, lipsticks, hair products (anti-dandruff), cosmetic gels (for their anti-irritant properties), cleansers (removal of scaly and calloused tissue), and moisturizers (increase water-binding capacity of the tissues).

From US—A—3 941 722 it is known that allantoine being an amphoteric compound forms "addition compounds" with various substances, e.g. with proteins. In this document a composition is disclosed comprising essentially three components, namely allantoin, a usual collagen protein and Crotein (a hydrolyzed collagen protein) which composition is readily soluble in water and is employed for skin treatments.

Until now, notwithstanding the acknowledged therapeutic qualities of allantoin and its derivatives including allantoin dl-panthenol modified, the amount of allantoin or allantoin equivalent contained in these compositions and employed in hydroalcoholic solutions (also referred to as aqueous-alcoholic solutions), such as after-shave lotions and colognes, has been severely restricted to relatively small amounts of 0.2% by weight or less. This is because when amounts of allantoin or allantoin dl-panthenol modified of greater than 0.25% by weight (based on the amount of allantoin) are employed in such hydroalcoholic solutions, the allantoin tends to crystallize out of solution within a relatively short period of time.

Accordingly, a long felt need exists in the market place for a stable form of allantoin which may be used in solution in amounts greater than 0.25% (based on the weight of allantoin) and upwards of 0.5% and more without crystallizing out of solution.

In accordance with the present invention, a complex or otherwise combined mixture of allantoin and hydrolyzed collagen-derived animal protein is provided which affords allantoin in a form which is easily solubilized in aqueous-alcoholic solutions in amounts of from 0.05% up to 5% and preferably from 0.5 to 2% based on the weight of allantoin. Such solutions may take the form of stable soothing hydroalcoholic skin preparations, such as colognes, after-shave lotions, skin toners, after bath splash and the like. The allantoin in the form of its complex or otherwise combined state, such as hydrogen bonding, with the hydrolyzed collagen-derived animal protein, will remain fully dissolved in the solution, without forming crystals, over extended periods of time.

The complex or otherwise combined allantoin and hydrolyzed collagen-derived animal protein product of the invention will contain the hydrolyzed collagen-derived animal protein in a weight ratio to the allantoin of within the range of from 100:1 to 1:1, preferably from 5:1 to 1:1, and optimally 1:1.

The method for preparing the complex of the invention, includes the steps of mixing allantoin and hydrolyzed collagen-derived animal protein in aqueous medium at a temperature of from about 45 to about 80°C, preferably from about 55 to about 75°C and more preferably from about 60 to about 65°C, for a period of from a few minutes (for example, 10 to 15 minutes) up to 1-1/2 hours or more to form a clear aqueous solution containing the complex. The clear aqueous solution will contain from 0.05 to 5% and preferably from 0.5 to 2% allantoin, and from 0.1 to 10% and preferably from 0.1 to 1.5% hydrolyzed collagen-derived animal protein.

The clear solution comprising the product of the invention may be used as such in the formation of any of the skin preparations mentioned or other products. If desired, the clear solution containing the complex of the invention may be dried, for example, freeze dried (or lyophilized) to form the complex or otherwise combined allantoin-hydrolyzed collagen-derived animal protein product in the form of a dry powder which may be subsequently added to water to form a solution or just simply added as a solid to powders, such as body and foot powders, emulsions, shampoos, acne products and the like.

The hydrolyzed collagen-derived animal protein has a molecular weight of within the range of from 100 to 200,000 and containing various amino acids including glycine, alanine, serine, threonine, proline, hydroxyproline, valine, isoleucine, phenylalanine, tyrosine, cystine/cysteine, methionine, aspartic acid, glutamic acid, arginine, histidine, lysine and hydroxylysine. Among the preferred hydrolyzed animal protein materials suitable for use herein are the collagen hydrolysates and derivatives referred to by the trademark Croteins manufactured by Croda, Inc., N.Y.C., Umordant sold by Pentapharm, Inc., Super-Pro 100 sold by Stepan Chemical Co., Proto-Lan 20 sold by Maybrook Inc., Lexein X-250 sold by Inolex Corp., Lanasan CL sold by Sandoz, Inc. and Peptein 2000 sold by Hormel. The Croda product is formed by hydrolyzing collagen (by alkali, acid or enzyme hydrolysis) and breaking the long collagen chains so that the molecular weight is reduced from the millions to hydrocolloids ranging from a molecular weight of 100 to 300,000 and preferably from 100 to 200,000. Amino acid composition of preferred collagen derived protein is set out below.

| Amino acid | % Present |
| --- | --- |
| Glycine | 20.0—30.5 |
| Alanine | 8.0—11.0 |
| Serine | 2.9—4.1 |
| Threonine | 1.8—2.6 |
| Proline | 13.7—18.0 |
| Hydroxyproline | 12.1—14.5 |
| Valine | 2.1—3.4 |
| Isoleucine | 1.3—1.8 |
| Leucine | 2.8—3.5 |
| Phenylalanine | 1.1—2.6 |
| Tyrosine | 0.2—1.0 |
| Cystine/Cysteine | 0.0—0.9 |
| Methionine | 0.7—0.9 |
| Aspartic acid | 5.7—9.0 |
| Glutamic acid | 10.0—11.7 |
| Arginine | 7.8—9.0 |
| Histidine | 0.7—1.0 |
| Lysine | 3.9—5.2 |
| Hydroxylysine | 0.7—1.2 |

As indicated, the combined allantoin-hydrolyzed collagen-derived animal protein product of the invention may be employed in a wide variety of products. A preferred product is a stable skin preparation which contains from 0.5 to 15% and preferably from 0.5 to 7% by weight of the complex or combined allantoin-hydrolyzed collagen-derived animal protein product. The stable skin preparation also contains panthenol, which serves as a skin moisturizer and humectant and is converted to an essential vitamin B-5 for the normal growth and toning of the tissue, present in an amount within the range of from 0.1 to 10% by weight, and preferably from 0.1 to 3% by weight; and urea, which functions as a skin conditioner, water-binding agent or skin softener present in an amount within the range of from 0.1 to 10%, and preferably from 0.1 to 5% by weight.

The above skin preparation will be in the form of an aqueous-alcoholic solution and as such will contain ethanol or isopropyl alcohol in an amount within the range of from 2.5 to 70%, and preferably from 10 to 30% by weight, and water in an amount within the range of from 20 to 80%, and preferably from 40 to 75% by weight. Thus, the aqueous-alcoholic solution will contain a weight ratio of water to alcohol of within the range of from 9:1 to 1:1, and preferably from 4:1 to 1:1.

The hydroalcoholic skin preparation of the invention may contain amounts of allantoin (greater than 0.5%) heretofore never retained in solution for extended periods of time. This apparently has been achieved by employing the allantoin in combined form with the hydrolyzed animal protein. It is theorized that the allantoin forms a complex with hydrolyzed animal protein which complex is substantially more soluble in the hydroalcoholic solution than is the allantoin by itself and so remains in solution for indeterminate periods. The result is that large amounts of allantoin (greater than 0.5%) may be employed to provide even more skin soothing preparations which remain stable over extended periods of time without having the allantoin crystallize out.

The stable soothing skin preparation containing the combined allantoin-hydrolyzed animal protein product may also comprise colognes, after-shave lotions, skin toners, emulsions, powders and the like. Accordingly, such skin preparations may include preservatives, examples of which include dimethyldimethoyl hydantoin, benzyl alcohol, imidazolidinyl urea, parabens and the like usually employed in amounts within the range of from 0.05 to 1.25% by weight, humectants, such as, sodium 2-pyrrolidone carboxylic acid, sorbitol, polyethylene glycols, propylene glycol, glycerine or other known humectants usually employed in amounts within the range of from 0.1 to 20% by weight, fragrances in amounts within the range of from 0 to 35%, and preferably from 0.1 to 20% by weight depending upon the ultimate use of the skin preparation; and solubilizing agents and emulsifiers for the fragrances, such as polyoxyethylene octyl phenyl ether and polyoxyethylene glycerine and other skin softeners, such as esters, lanolin, lanolin derivatives thereof and/or other conventional skin softeners, present in an amount within the range of from 0.1 to 10% by weight, and color as deemed necessary.

The skin preparation may also include therapeutic agents, such as benzoyl peroxide, methyl salicylate or sun-screens in amounts normally employed for the particular therapeutic agent present.

Preferred formulations within the scope of the present invention contain from 0.6 to 3.5% by weight combined allantoin-collagen-derived animal protein, from 0.1 to 3% by weight d- or dl-panthenol, from 0.1 to 5% by weight urea, preservatives, such as benzyl alcohol, imidazolidinyl urea and/or dimethyldimethoyl hydantoin, humectants such as sodium-2-pyrrolidone carboxylic acid, emulsifiers, such as polyoxyethylene octyl phenyl ether, feel enhancers, such as polyoxyethylene glycerine, fragrance, water and ethanol or isopropyl alcohol.

0 087 374

The skin preparations containing the combined allantoin-hydrolyzed animal protein product may be prepared as follows.

Ethanol or isopropyl alcohol, and where present, preservatives such as benzyl alcohol, fragrance oils, and emulsifiers and solubilizing agents for the fragrance oils are mixed together to form a first mixture.

Allantoin, hydrolyzed animal protein, d- or dl-panthenol, urea, and, where present, humectants, preservatives such as dimethyldimethoyl hydantoin and water are mixed together to form a second mixture containing the allantoin-hydrolyzed animal protein combined product. The second mixture is heated to about 60 to 65°C or until clear, and then cooled to about 25°C.

The second mixture is added to the first mixture with agitation and the mixture is allowed to age for 2 to 4 days. The mixture is then chilled at 0 to 4°C, filtered and coloring solution is added to form the skin preparation of the invention.

The following Examples represent preferred embodiments of the invention.

Example 1

A combined allantoin-hydrolyzed animal protein product in accordance with the invention was prepared as described below.

0.5 Grams allantoin and 0.5 gm hydrolyzed collagen-derived animal protein (Crotein® SPA) are mixed with 66 gm of water and the mixture was heated at 60 to 65°C for about 10 minutes until a clear solution forms. The mixture is then allowed to cool to room temperature and is then ready for use as an additive in colognes, after-shave lotions, skin toners, shampoos, acne products, emulsions, and the like.

If desired, the above solution may be freeze-dried to form a concentrate or powder comprising the combined allantoin-hydrolyzed collagen-animal protein which may be used in powder compositions such as body and foot powders, various cosmetics, and the like.

Example 2

A stable soothing cologne having the following composition was prepared as described below.

| Ingredient | Parts by weight |
| --- | --- |
| Mix A | |
| Ethanol (denatured alcohol SDA 40 Reg) | 20 |
| Benzyl alcohol | 0.5 |
| Polyoxyethylene (13) octyl phenyl ether (Triton® X-102) | 7 |
| Polyoxyethylene (26) glycerine (Liponic® EG-1) | 0.5 |
| Fragrance | 3.3 |
| | |
| Mix B | |
| Allantoin | 0.5 |
| Hydrolyzed animal protein (Crotein® SPA) | 0.5 |
| dl-Panthenol | 1 |
| Na-2-pyrrolidone carboxylic acid (Ajidew®-N-50) | 0.2 |
| Urea | 0.5 |
| Dimethyldimethoyl hydantoin (55%) (Glydant) | 0.2 |
| Deionized water | 66 |
| Color (FD&C Blue #1—0.1% aqueous solution) | 0.2 |

The ingredients identified by Mix A were mixed together to form a first mixture. The ingredients identified by Mix B were mixed together to form a second mixture which was heated to 60—65°C until a clear solution formed and then cooled to room temperature. The first mixture was then added to the second mixture (containing the combined allantoin-hydrolyzed animal protein product) with stirring and the combined mixture was then chilled to 0—4°C, and filtered through a #7 Ertel pad. The color solution was then added to form the soothing cologne of the invention wherein the allantoin remained stable and in solution, without crystallizing out, over extended periods of several months.

4

Example 3

A stable soothing after-shave lotion having the following composition was prepared as described in Example 1.

| Ingredient | Parts by weight |
|---|---|
| **Mix A** | |
| Ethanol (denatured alcohol SDA 40 Reg) | 20 |
| Benzyl alcohol | 0.5 |
| Polyoxyethylene (13) octyl phenyl ether (Triton® X-102) | 4 |
| Polyoxyethylene (26) glycerine (Liponic® EG-1) | 0.3 |
| Fragrance | 2 |
| **Mix B** | |
| Allantoin | 0.5 |
| Hydrolyzed animal protein (Crotein® SPA) | 0.5 |
| dl-Panthenol | 1 |
| Na-2-pyrrolidone-5-carboxylate (Ajidew®-N-50) | 0.5 |
| Urea | 0.5 |
| Dimethyldimethoyl hydantoin (55%) (Glydant) | 0.2 |
| Deionized water | 70 |
| FD&C Blue #1 (0.1% aqueous solution) | 0.1 |

The after-shave lotion produced as described above was found to have excellent stability with the allantoin remaining in solution, without crystallizing out, over extended periods of time.

**Claims**

1. A stable soothing hydroalcoholic skin preparation comprising a combined allantoin-hydrolyzed collagen-derived animal protein product in an amount of at least 0.5% by weight, and panthenol, urea, ethanol or isopropyl alcohol and water, and optionally one or more humectants, preservatives, fragrances and solubilizing agents for the fragrances, said allantoin remaining in solution, without crystallizing out, for extended periods of time.

2. The skin preparation as defined in Claim 1 wherein said hydrolyzed collagen-derived animal protein is present in a weight ratio to the allantoin of within the range of from 100:1 to 1:1.

3. The skin preparation as defined in Claim 1 in the form of an aqueous solution containing from 0.05 to 5%, particularly from 0.5 to 2%, by weight allantoin and from 0.1 to 10%, particularly from 0.1 to 1.5%, by weight hydrolyzed collagen-derived animal protein.

4. The skin preparation as defined in Claim 1 wherein the hydrolyzed collagen-derived animal protein has a molecular weight within the range of from 100 to 300,000.

5. The skin preparation as defined in Claim 1 wherein the panthenol is in the d- or dl-form and is present in an amount within the range of from 0.1 to 10% by weight.

6. The skin preparation as defined in Claim 1 wherein the urea is present in an amount within the range of from 0.1 to 10% by weight.

7. The skin preparation as defined in Claim 1 further including a preservative which is dimethyldimethoyl hydantoin, imidazolidinyl urea, benzyl alcohol or a paraben.

8. The skin preparation is defined in Claim 1 further including a humectant which is propylene glycol, a polyethylene glycol, sorbitol, glycerine, sodium-2-pyrrolidone carboxylic acid or a mixture thereof.

9. The skin preparation as defined in Claim 1 further including a fragrance and a solubilizing agent for the fragrance which is polyoxyethyene octyl phenyl ether.

10. The skin preparation as defined in Claim 1 wherein the water is present in an amount within the range of from 20 to 80% by weight and the alcohol is present in an amount within the range of from 2.5 to 50% by weight, and the water to alcohol weight ratio is from 1:1 to 9:1.

11. The skin preparation as defined in Claim 1 in the form of a cologne, after-shave lotion, skin toner, or emulsion.

**Patentansprüche**

1. Stabiles beruhigendes hydroalkoholishes Hautpräparat umfassend ein Kombinationsprodukt aus Allantoin und von hydrolysiertem Kollagen abgeleitetem tierischen Protein in einer Menge von mindestens 0,5 Gewichtsprozent und Panthenol, Harnstoff, Äthanol oder Isopropanol und Wasser und gegebenenfalls ein oder mehrere Feuchthaltemittel, Konservierungsmittel, Duftstoffe und Lösungsvermittler für die Duftstoffe, wobei das Allantoin ohne auszukristallisieren über längere Zeiträume in Lösung verbleibt.

5

2. Hautpräparat nach Anspruch 1, in dem das von hydrolysiertem Kollagen abgeleitet tierische Protein in einem Gewichtsverhältnis zum Allantoin im Bereich von 100:1 bis 1:1 vorhanden ist.

3. Hautpräparat nach Anspruch 1, in Form einer wäßrigen Lösung mit einem Gehalt von 0,05 bis 5, insbesondere von 0,5 bis 2 Gewichtsprozent Allantoin und 0,1 bis 10, insbesondere 0,1 bis 1,5 Gewichtsprozent von hydrolysiertem Kollagen abgeleitetes tierisches Protein.

4. Hautpräparat nach Anspruch 1, in dem das von hydrolysiertem Kollagen abgeleitete tierische Protein ein Kolekulargewicht im Bereich von 100 bis 300 000 aufweist.

5. Hautpräparat nach Anspruch 1, in dem das Panthenol in der d- oder dl-Form und in einer Menge im Bereich von 0,1 bis 10 Gewichtsprozent vorliegt.

6. Hautpräparat nach Anspruch 1, in dem der Harnstoff in einer Menge im Bereich von 0,1 bis 10 Gewichtsprozent vorliegt.

7. Hautpräparat nach Anspruch 1 mit einem zusätzlichen Gehalt an einem Konservierungsmittel, welches Dimethyldimethoylhydantoin, Imidazolidinylharnstoff, Benzylalkohol oder ein Paraben ist.

8. Hautpräparat nach Anspruch 1 mit einem zusätzlichen Gehalt an einem Feuchthaltemittel, welches Propylenglykol, ein Polyäthylenglykol, Sorbit, Glycerin, Natrium-2-pyrrolidoncarbonsäure oder ein Gemisch davon ist.

9. Hautpräparat nach Anspruch 1 mit einem zusätzlichen Gehalt an einem Duftstoff und einem Lösungsvermittler für den Duftstoff, welcher Polyoxyäthylenoctylphenyläther ist.

10. Hautpräparat nach Anspruch 1, in dem das Wasser in einer Menge im Bereich von 20 bis 80 Gewichtsprozent und der Alkohol in einer Menge im Bereich von 2,5 bis 5 Gewichtsprozent vorliegen und das Gewichtsverhältnis von Wasser zu Alkohol von 1:1 bis 9:1 beträgt.

11. Hautpräparat nach Anspruch 1 in Form eines Kölnisch-Wasser, einer After-Shave Lotion, eines Haut-Toners oder einer Emulsion.

## Revendications

1. Préparation hydro-alcoolique calmante stable pour la peau, comprenant une combinaison d'allantoïne et de protéine animale dérivée du collagène et hydrolysée, en quantité d'au moins 0,5% en poids, et du panthénol, de l'urée, de l'éthanol ou de l'alcool isopropylique et de l'eau, et facultativement un ou plusieurs humidifiants, conservateurs, parfums et agents solubilisants pour les parfums, ladite allantoïne restant en solution, sans crisalliser, pendant des laps de temps prolongés.

2. Préparation pour la peau selon la revendication 1, dans laquelle ladite protéine animal dérivée du collagène et hydrolysée est présente dans un rapport pondéral à l'allantoïne qui est situé dans l'intervalle de 100:1 à 1:1.

3. Préparation pour la peau selon la revendication 1, sous la forme d'une solution aqueuse contenant de 0,05 à 5%, en particulier de 0,5 à 2%, en poids, d'allantoïne, et de 0,1 à 10%, en particulier de 0,1 à 1,5%, en poids, de protéine animal dérivée du collagène et hydrolysée.

4. Préparation pour la peau selon la revendication 1, dans laquelle la protéine animal dérivée du collagène et hydrolysée a un poids moléculaire compris dans l'intervalle de 100 à 300,000.

5. Préparation pour la peau selon la revendication 1, dans laquelle le panténol est sous la forme d- ou dl- et est présent en quantité comprise dans l'intervalle de 0,1 à 10' en poids.

6. Préparation pour la peau selon la revendication 1, dans laquelle l'urée est présente en quantité comprise dans l'intervalle de 0,1 à 10% en poids.

7. Préparation pour la peau selon la revendication 1, contenant en outre un conservateur qui est la diméthyl diméthoyl hydantoïne, l'imidazolidinyl urée, l'alcool benzylique ou un para-hydroxybenzoate.

8. Préparation pour la peau selon la revendication 1, contenant en outre un humidifiant qui est le propylène glycol, un polyéthylène glycol, le sorbitol, la glycérine, l'acide 2-pyrrolidone carboxylique sodique ou un mélange des précédents.

9. Préparation pour la peau selon la revendication 1, contenant en outre un parfum, et un agent solubilisant pour ce parfum, qui est le polyoxyéthylène octyl phényl éther.

10. Préparation pour la peau selon la revendication 1, dans laquelle l'eau est présente en quantité comprise de 20 à 80% en poids, et l'alcool est présent en quantité comprise dans l'intervalle de 2,5 à 50% en poids, le rapport pondéral eau sur alcool étant de 1:1 à 9:1.

11. Préparation pour la peau selon la revendication 1, sous la forme d'une eau de Cologne, d'une lotion d'après-rasage, d'un tonique pour la peau, ou d'une émulsion.